(19) Europäisches Patentamt
European Patent Office
Office européen des brevets

(11) **EP 4 082 422 A1**

(12) **EUROPEAN PATENT APPLICATION**

(43) Date of publication:
**02.11.2022 Bulletin 2022/44**

(51) International Patent Classification (IPC):
**A61B 3/028** *(2006.01)* **A61B 3/032** *(2006.01)*

(21) Application number: **21305547.8**

(22) Date of filing: **28.04.2021**

(52) Cooperative Patent Classification (CPC):
**A61B 3/0285; A61B 3/032**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **ESSILOR INTERNATIONAL**
**94220 Charenton-Le-Pont (FR)**

(72) Inventors:
• **HERNANDEZ-CASTANEDA, Martha**
**94220 Charenton (FR)**

• **PETIGNAUD, Cécile**
**94800 Villejuif (FR)**
• **MARIN, Gildas**
**75012 Paris (FR)**
• **BAILLON, Loïc**
**75011 Paris (FR)**
• **PERROT, Stéphane**
**94100 saint Maur des Fossés (FR)**
• **ROUSSEAU, Denis**
**38610 GIERES (FR)**

(74) Representative: **Jacobacci Coralis Harle**
**32, rue de l'Arcade**
**75008 Paris (FR)**

(54) **OPTOMETRY DEVICE FOR BINOCULAR TESTING OF THE EYES OF A SUBJECT**

(57) The invention relates to an optometry device (1, 2) for binocular testing of the eyes of a subject, comprising:
- a binocular refraction test unit (10) with a first optical refraction element (11) adapted to provide different vision correction powers along a first optical axis (OA1) and a second optical refraction element (12) adapted to provide different vision correction powers along a second optical axis (OA2);
- a display system (20) for providing a first and a second test images (I11; I12; I21; I22), the first test image being conveyed to the first optical refraction element (11) along a first optical pathway (OP1) and the second test image being conveyed to the second optical refraction element (12) along a second optical pathway (OP2);

said first and second test image being provided with details smaller than 1 arc minute, over a field of view of at least 8° in horizontal directions,
wherein said first and second optical pathways (OP1, OP2) have a length comprised between 25 and 100 centimeters, allowing testing the eyes of the subject in near and/or intermediary vision.

**Fig.1**

EP 4 082 422 A1

**Description**

TECHNICAL FIELD OF THE INVENTION

The invention relates to an optometry device for binocular testing of the eyes of a subject.

The device according to the invention may be in particular used for testing the eyes of the subject in near and/or intermediary vision conditions, with natural or ergonomic and comfortable conditions that may be precisely controlled.

BACKGROUND INFORMATION AND PRIOR ART

Known devices and methods for binocular testing of the eyes of a subject are usually dedicated to testing the eyes in far vision conditions. In far vision conditions, the image shown to the subject to test his binocular far vision is usually placed at over 1 meter from the subject's eyes.

However, the refraction features of the eyes in near and/or intermediary vision conditions may be different from the refraction features in far vision. This may be due, among other things, to the fact that the accommodation and convergence of the eyes are different in near and/or intermediary vision conditions.

Known methods for testing the eyes of a subject in near and/or intermediary vision are implemented with visual targets ensuring a straight forward gaze direction of the subject, or with the subject wearing trial frames, leading to non natural posture of near and/or intermediary vision. The conditions of the near and/or intermediary vision test are not comfortable for the patient and not easy to control for the eye care practitioner.

These known methods simulate the visual compensation to be provided, for example by means of a refractometer (also called phoropter).

Such a phoropter comprises a refraction test unit and a support element designed to receive the head of the individual and to hold it in a predetermined position relative to the refraction test unit.

This refraction test unit houses trial lenses providing different corrections that can be successively placed in front of the individual's eyes until a suitable correction is found.

In the phoropter, the trial lenses are situated on two discs that are mounted so as to be free to rotate. Consequently, the trial lenses of this disc can successively be positioned in front of the corresponding patient's eyes.

Such a phoropter only permits measurement straight ahead and is not able to precisely control the binocular vision and accommodation of the individual.

It is known that the required visual correction is not the same in near vision with horizontal gaze and with downward gaze.

It is also known that accommodation is not the same in binocular vision and in monocular vision, and that the accommodation changes as a function of gaze orientation and of the used vision (monocular or binocular).

SUMMARY OF THE INVENTION

Therefore one object of the invention is to provide a new device allowing determining precisely the binocular refraction features of the eyes of the subject in near and/or intermediary vision conditions.

The above object is achieved according to the invention by providing an optometry device for binocular testing of the eyes of a subject, comprising:

- a binocular refraction test unit with a first optical refraction element adapted to provide different vision correction powers along a first optical axis and a second optical refraction element adapted to provide different vision correction powers along a second optical axis;
- a display system for providing a first and a second test images, the first test image being conveyed to the first optical refraction element along a first optical pathway and the second test image being conveyed to the second optical refraction element along a second optical pathway;

said first and second test image being provided with details smaller than 1 arc minute, over a field of view of at least 8° in horizontal directions, wherein said first and second optical pathways have a length comprised between 25 and 100 centimeters, allowing testing the eyes of the subject in near and/or intermediary vision.

Preferably, in order to determine accurately the refraction features of the eyes of the subject in near and/or intermediary vision, the device according to the invention is such that said binocular refraction test unit presents an inclined vision test configuration where the first and second optical axes of the first and second refraction elements are inclined downwards. Satisfactory ergonomic conditions for determining the refraction features in near and/or intermediary vision are therefore provided.

A further object of the invention is to provide a device allowing determining precisely the binocular refraction features of the eyes of the subject both in a horizontal vision configuration and in an inclined vision configuration with a simple and quick way of switching from one configuration to the other. This allows in particular testing the eyes in far and near and/or intermediary vision conditions with a simple way of changing the conditions from near or intermediary vision to far vision and from far vision to near vision or intermediary vision.

To this end, said display system is preferably mechanically linked to said binocular refraction test unit to be integral with the binocular refraction test unit.

Moreover, said binocular refraction test unit is advantageously mobile between at least two test configurations: a horizontal vision test configuration where the first and second optical refraction elements are positioned in a first position where said first and second optical axis extend horizontally and an inclined vision test configuration where the first and second optical refraction elements are inclined compared to said first position, so that said first and second optical axis are inclined downwards.

Preferably, in said optometry device according to the invention, said binocular refraction test unit is mobile as a whole in rotation about a horizontal rotation axis parallel to a mean plane of the first and second optical refraction elements, said horizontal rotation axis going through a predetermined point located where a center of rotation of one of the eyes of the subject is situated when the subject's eyes look through the refraction test elements.

It is then possible to switch from a horizontal vision test to an inclined vision test easily, without having to check twice the alignment of the eyes of the subject with the optical pathways of the light emitted by said first and second test images.

According to other advantageous and non limiting features of the device according to the invention :

- the position of said first and second test images within said display system is adjustable in order for said first and second test images to be aligned with said first or second optical pathways;
- said device comprises an alignment verification device for checking the alignment of said first and second optical pathways with each eye of the subject, comprising at least a camera oriented towards the eyes of the subject or an eyetracking device;
- said display system of said device may be moved forward or backward towards the subject or away from the subject to determine the amplitude of the sharpness of the vision of the subject with a direct measurement of this amplitude;
- said first and second test images provided by said display system comprise images of realistic activities carried out in near vision or intermediary vision having a plurality of stereoscopic peripheral image components, adapted to be seen in three dimensions by the subject viewing each of said test images with one of his eyes;
- at least two image components of said plurality of stereoscopic image components are displayed with different values of binocular disparity;
- said display system comprises a single display zone where said first and second test images are both produced and an image selection unit for selectively conveying said first and second test images to the first or second optical refraction element along said first or second optical pathway;
- said image selection unit comprises a filter, adapted to transmit and/or reflect selectively light having a wavelength comprised in a predetermined wavelength range or a polarizer, adapted to transmit and/or reflect selectively light having a polarization oriented along a predetermined axis; in this case, said display system produces said first and second test images preferably simultaneously in said display zone;
- said display system produces said first and second test images simultaneously in said display zone and said image selection unit comprises a plurality of microlenses, each of them mapped with a part of the single display zone and adapted to convey the light emitted by this part of the single display zone in at least two different directions;
- said display system produces said first and second test images alternatively in said single display zone;
- said image selection unit comprises two shutters, each of them being associated with one of said first and second optical pathways and presenting two states: an activated state in which each shutter blocks selectively the propagation of light in the corresponding first or second optical pathway, and a deactivated state in which each shutter allows selectively the propagation of light in the same first or second optical pathway;
- said device comprises a synchronization device provided to synchronize the change of state of both shutters and the production of the first and second test images;
- each shutter comprises an electronic shutter associated with the display system and/or with the refraction test unit;
- said display system produces said first and second test images in two separated display zones and wherein said optometry device comprises a light conveying unit conveying said first and second test images to said first and second eye along said first and second pathways;
- said light conveying unit comprises a polarizer rotator which rotates alternatively in front said first and second test images;
- said light conveying unit comprises a wall blocking the transmission of light between said first and second optical pathways;
- said light conveying unit comprises a mobile polarizer or a variable polarizer;
- said display system comprises two separate screens, each of said separate screens displaying one of said first and second test images;
- said light conveying unit comprises a beam splitter;
- said display system comprises:

  - two separate microdisplays, each of said separate microdisplays displaying one of said first and second test images or
  - one screen providing said two separated display zones, each of said separated display zones displaying one of said first and second test images;

- said light conveying unit comprises two optical guides, each conveying the light emitted at one of said separated display zones towards one of the eye of the subject.

DETAILED DESCRIPTION OF EXAMPLE(S)

The following description with reference to the accompanying drawings will make it clear what the invention consists of and how it can be achieved. The invention is not limited to the embodiments illustrated in the drawings. Accordingly, it should be understood that where features mentioned in the claims are followed by reference signs, such signs are included solely for the purpose of enhancing the intelligibility of the claims and are in no way limiting on the scope of the claims.

In the appended drawings:

- figure 1 is a schematic view of a first embodiment of the optometry device according to the invention, in an inclined vision configuration for binocular testing of the eyes of a subject,
- figure 2 is a schematic view of the optometry device of figure 1, in a horizontal vision configuration for binocular testing of the eyes of a subject,
- figure 3 is a schematic view of a second embodiment of the optometry device according to the invention, in an inclined vision configuration for binocular testing of the eyes of a subject,
- figures 4 to 6 are schematic views of a refraction test unit for the optometry device of figure 1 or 2, in front of the eyes of a subject in three different positions: a position of horizontal gaze through the refraction test unit (figure 4), a position of inclined gaze through the refraction test unit (figure 6), and an intermediary position of the refraction test unit (figure 5);
- figure 7 and 8 are schematic views of different examples of implementation of a refraction test unit for the optometry device of figure 1 or 2, wherein said refraction test unit is configured to pivot about a horizontal rotation axis going through a predetermined point, said point being in particular a center of rotation of one of the eye of the subject,
- figure 9 is a schematic top view of a first example of implementation of a display system for the optometry device of figure 1 or 2,
- figure 10 is a schematic top view of a second example of implementation of a display system for the optometry device of figure 1 or 2,
- figures 11, 12 and 13 are schematic partial views of the display system shown on figure 9,
- figure 14 is a schematic front view of a screen of a third example of implementation of a display system for the optometry device of figure 1 or 2,
- figure 15 is a schematic side sectional view of the screen of figure 14,
- figure 16 is a schematic representation of the synchronization data of a screen and two shutters of a display system in a fourth example of implementation of the optometry device,
- figure 17 is a schematic top view of said fourth example of implementation of a display system for the optometry device of figure 1 or 2,
- figures 18 and 20 are examples of test images displayed by the display system of the optometry device of figure 1 or 2,
- figures 19 and 21 are examples of images seen in binocular vision by the subject when presented with the images represented on figures 18 and 20.

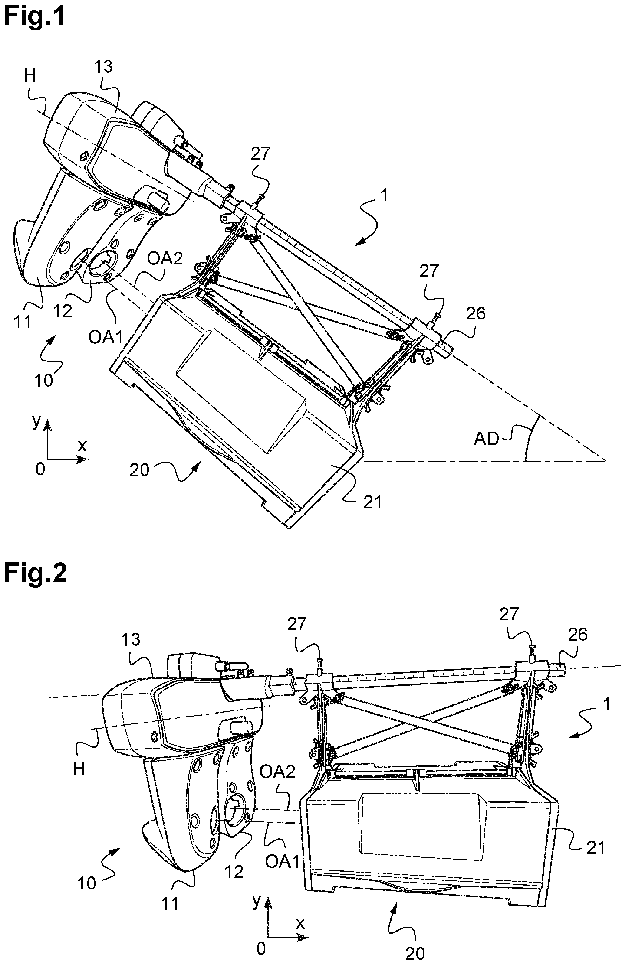

Figure 1, 2, 3 and 13 represents schematically, in perspective, the main elements of two embodiments of a binocular optometry device 1; 2 for determining at least one refraction feature of a first eye E1 and/or of a second eye E2 of a subject, in a binocular manner.

This binocular determination of the refraction feature of an eye is based on a binocular measurement performed while the subject has both eyes E1, E2 opened and un-obstructed.

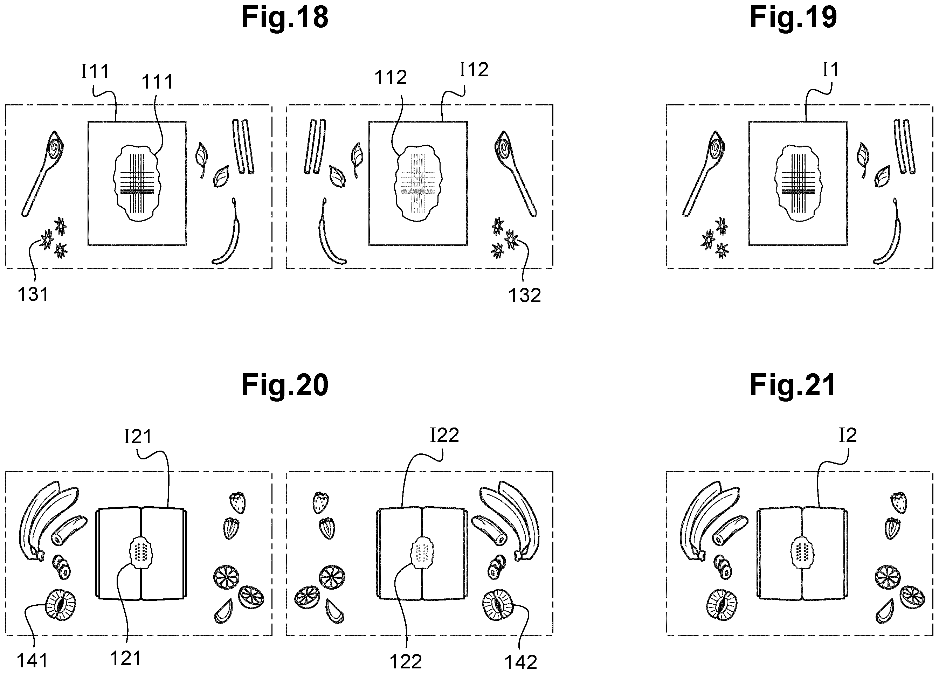

More precisely, during said binocular measurement, each eye E1, E2 of the subject is provided with a test image. The test images provided to both eyes are configured such that the fusion of the two test images by the brain of the subject may occur. Preferably, the two test images are stereoscopic images providing a representation I1, I2 (figures 19 and 21) at least partially in three dimensions for the subject.

In order to achieve this end, each test image is configured to be accurately aligned with the corresponding eye of the subject.

The optometry device 1, 2 according to the invention comprises a binocular refraction test unit 10 with a first optical refraction element 11 adapted to provide different vision correction powers along a first optical axis OA1 and a second optical refraction element 12 adapted to provide different vision correction powers along a second optical axis OA2 (figures 1 to 6 and 11).

Said first optical refraction element 11 is configured for providing the first eye E1 of the subject with a first correction power, and said second optical refraction element 12 is configured for providing the second eye E2 of the subject with a second correction power.

By correction power, it is meant a dioptric power allowing correcting a refraction error of the eye of the subject, such as sphere power, cylinder power and axis, prismatic power and axis...

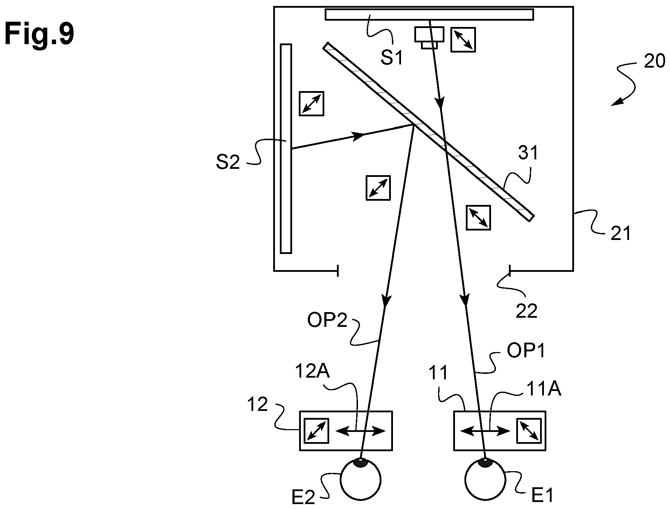

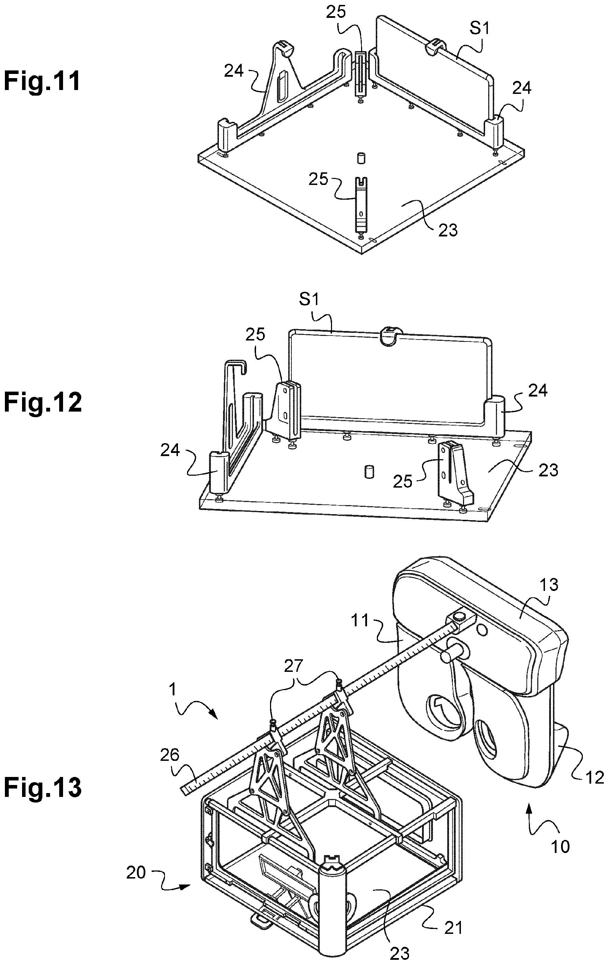

It also comprises a display system 20 for providing a first and a second test images, the first test image being conveyed to the first optical refraction element 11 along a first optical pathway OP1 and the second test image being conveyed to the second optical refraction element 12 along a second optical pathway OP2 (figures 9 et 11).

The image display system 20 is thus configured for providing the first test image I11, I21 to the first eye E1 of a subject and, at the same time, for providing the second test image 112, 122 to the second eye E2 of the subject, the second test image being different from the first test image.

The first test image 111, 121 is seen by the first eye E1 of the subject through the first optical refraction element 11, while the second test image 112, 122 is seen by the second eye E2 of the subject through the second optical refraction element 12.

In a remarkable way, said first and second test images are provided with details smaller than 1 arc minute, over a field of view of at least 8° in horizontal directions and said first and second optical pathways OP1, OP2 have a length comprised between 25 and 100 centimeters, allowing testing the eyes of the subject in near and/or intermediary vision.

Each of the first and second optical refraction elements 11, 12 comprises a lens or a mirror or a prism, or a set of such optical components that has adjustable refractive power features or permits to adjust refractive power by rotation or translation of the optical components.

The length of each optical pathway is measured from the test image displayed to the outside face of the lens, prism or set of optical components of the optical refraction element intended to be turned towards the eye of the subject. In practice, it is for example equal here to the distance along which the light is conveyed from the screen to the outside face of the set of optical components.

In the embodiments of the optometry device 1, 2 shown on the appended figures, and described in the following, each of said first and second optical refraction elements 11, 12 comprises a lens with variable power. It comprises here a deformable liquid lens having an adjustable shape. The optical axis OA1, OA2 mentioned before thus correspond to the optical axis of the corresponding lens.

Alternatively, or in addition, the optical refraction element may comprise an ensemble of non-deformable lenses having different optical powers, and a mechanical system that enables to select some of these lenses to group them to form the set of lenses through which the subject can look. In this last case, to adjust the refractive power of the set of lenses, one or several lenses of the set of lenses are replaced by other lenses stored in the refraction test unit. The optical axis mentioned before thus corresponds to the optical axis of the lens placed in front of the eye of the subject.

In particular, each optical refraction element 11, 12 may comprise a lens with variable spherical power and an optical component with a variable cylindrical power and variable cylindrical axis. It may also comprise an optical component adapted to provide prismatic power to the eyes of the subject. The resulting set of optical components is schematically represented on the figures by a lens 11A, 12A.

Each of the optical refraction elements 11, 12 is intended to be placed in front of one of the eyes 2, 3 of the subject, close to this eye. It is placed, for example, at a distance comprised between 5 and 25 millimeters from the eye. The distance is for example measured between the outside face of the set of optical components 11A, 12A of the optical refraction element turned towards the eye and the eye placed in front of it, between the apex of the curvature of the outside face of the set of optical components 11A, 12A and the apex of the curvature of the cornea of the eye.

Each of the eye E1, E2 of the subject can see said first or second test image 111, 112; 121, 122 displayed by said display system 20 through the set of optical components of the optical refraction element 11, 12, through the lens or through the set of lenses, or by reflection onto a mirror of the optical refraction element in an alternative implementation.

In the following, we will describe the optometry device in the case where each optical refraction element comprises a set of optical components 11A, 12A with a lens with variable spherical power and a optical component with a variable cylindrical power and variable cylindrical axis and, optionally, a prism, represented on the figures by the lens with the reference 11A, 12A.

The optical set has an overall spherical power S corresponding to the spherical optical power, expressed in diopters. The optical set has a cylindrical power C expressed in diopters and an orientation represented by an angle A. Each of the first and second refraction correction, provided by the corresponding optical refraction elements 11, 12, may be characterized by the values of these three refractive power parameters S, C and A.

Said optical refraction elements 11, 12 are mounted on a common support 13 that extends between the optical refraction elements, above them, along a longitudinal axis H (figures 1 to 3) that is horizontal.

This support 13 is linked to a global supporting structure (not represented or only partially represented on the figures) that lies on a table or on the ground.

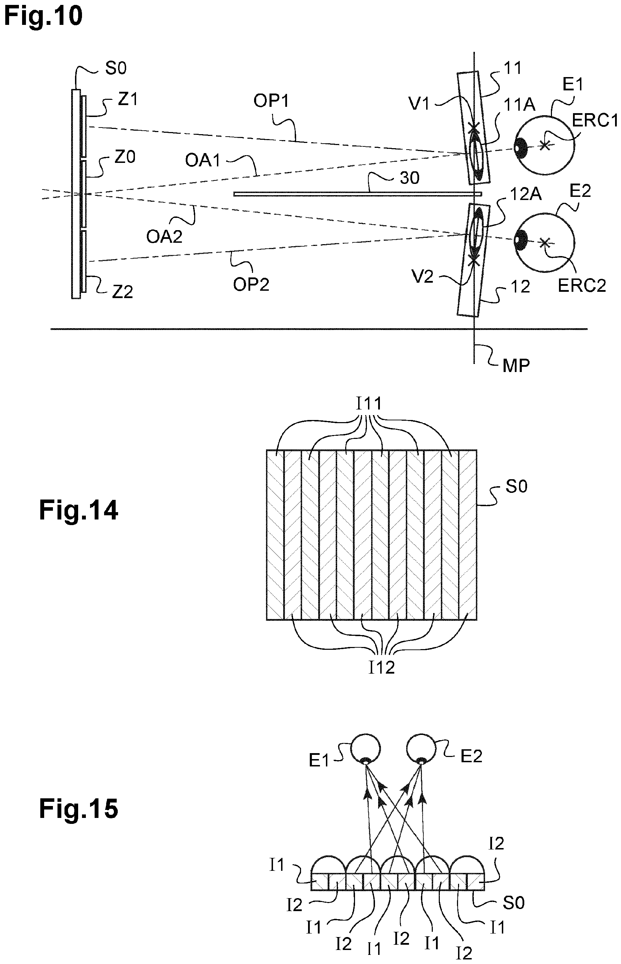

As described later, each of the optical refraction elements 11, 12 is mounted on the support 13 to be mobile in rotation about an axis V1, V2 perpendicular to said longitudinal axis H. A mean plane MP of the first and second optical refraction elements 11, 12 goes through these axes V1, V2 (figure 10). As shown on figure 10, this mobility of the optical refraction elements allows adjusting the alignment of the optical axes OA1, OA2 with the eyes of the subject taking into account their convergence in near and/or intermediary vision conditions.

Preferably, each axis V1, V2 about which one of said optical refraction elements is mobile in rotation go through a predetermined point located where a center of rotation of the corresponding eye of the subject is situated when the subject's eyes look through the refraction test elements (not shown on the figures). This allows keeping a centered gaze into the optical refraction elements without modifying the distance between the two optical refraction elements when going from a far vision test (without convergence) to a near and/or intermediary vision test or from near and/or intermediary vision to far vision test.

Said display system 20 is adapted to provide said first and second test images, examples of which will be provided hereafter, with details smaller than 1 arc minute, over a field of view of at least 8° in horizontal directions, in near and/or intermediary vision conditions.

It is considered here that near and intermediary vision conditions encompass the vision of a target placed at a distance corresponding to an optical pathway comprised between 25 and 100 centimeters between the optical refraction element 11, 12 and the test images displayed by the display system 20 plus the distance between the optical refraction element and the eye .

This distance may be fixed or variable. The variation of the distance between the test images displayed by the display system and the optical refraction elements may be obtained by varying the distance between the display system as a whole and the refraction test unit or by using mobile optical components such as mirrors inside the display system 20.

In the case where the distance between the test images displayed by the display system and the optical refraction elements is variable, it can be varied over a wider range, for example 25 cm to 6 or 8 meters or to infinity. The optometry device according to the invention may then be used to test the vision of the subject in far vision conditions or in near or in intermediary vision conditions.

The test images having details smaller than 1 arc minute allows testing visual acuity of the subject better than 10 tenth (10/10th) or 0 logMAR, ideally up to 15/10th (-0.18logMAR) or even 20/10th (-0.3logMAR).

In order to obtain such test images, said display system may comprise at least one screen with pixel having a size that allows displaying images to measure a visual acuity of 15/10th or 20/10th, for example having a size comprised between 80 to 20 micrometers

The use of a field of view of at least 8° in a horizontal direction ensures an easier accommodation and fusion, and thus improves the comfort of the subject while better controlling the near vision conditions. Preferably, the field of view of the display system 20 is of 8° in vertical and horizontal directions.

In order to obtain such test images, said display system may comprise at least one screen with a zone of display for each test image having dimensions adapted to give such field of view. Dimensions of at least of 56 millimeters for a viewing distance of 40 centimeters (cm) will give the field of view of 8°. Preferably, screens with dimensions comprised between 35 to 147 millimeters may be used for viewing distances respectively comprised between 25 to 105 centimeters.

Advantageously, here, said refraction test unit presents an inclined vision test configuration where the first and second optical axes of the first and second refraction elements are inclined downwards.

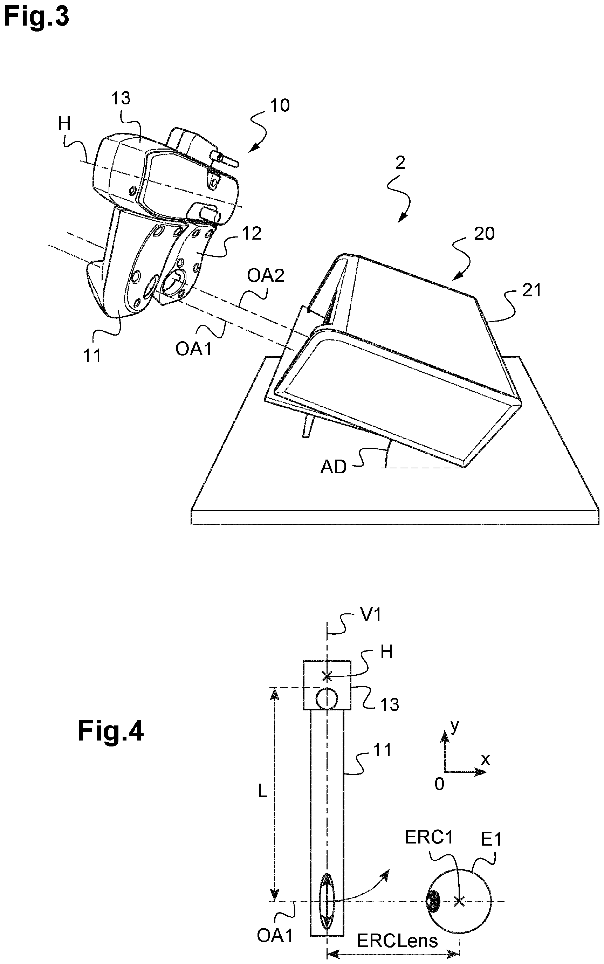

For example, the optical axes are inclined of an angle AD relative to a horizontal plane (figures 2 and 3). This angle AD is preferably comprised between 10 and 50 degrees. This allows providing an ergonomic near vision posture for the subject when testing his eyes in near vision conditions. In this ergonomic near vision posture, the subject gazes downwards through the optical refraction elements.

This ergonomic near vision posture is for example close to the natural reading posture of the subject.

The values of the refraction features determined for near vision are more accurate when the subject is placed in such an ergonomic position. Accommodation and convergence of the subject are indeed close to the accommodation and convergence in a natural near vision situation such as reading.

This inclined vision test configuration of the optometry device 1, 2 according to the invention may be obtained in different manners.

In the first preferred embodiment of figures 1 and 2, said display system 20 is mechanically linked to said binocular refraction test unit 10 to be integral with the binocular refraction test unit 10.

In other words, said display system 20 and said binocular refraction test unit 10 are attached so that they can only move together, with no possible relative movements.

This attachment may be permanent. Alternatively, the display system 20 and binocular refraction test unit 10 may be uncoupled in a transitory period in order to modify their relative position before performing the visual tests.

For example, as described hereafter, in some embodiments the distance between said display system 20 and said refraction test unit 10 may be modified whereas they can only pivot together as one piece.

As shown on figures 1 and 2 and described hereafter in more details, the display system 20 is housed in a box 21 mounted on a rail 26. Said rail 26 is attached to said common support 13 for the optical refraction element 11, 12. The rail 26 extends globally perpendicularly to the mean plane of said optical refraction element 11, 12.

In the second embodiment of figure 3, said display system 20 is independent and separate from the binocular refraction test unit 10. The display system 20 is placed on a horizontal surface, such as a table, in a fixed position where the optical pathways OP1, OP2 are inclined relative to the horizontal surface by said angle AD. The display system 20 of the second embodiment is for example similar to the display system of the first embodiment, except that it is not mounted on a rail but placed on a horizontal surface.

In both embodiments, said binocular refraction test unit 10 is mobile as a whole in rotation about a horizontal rotation axis parallel to the mean plane of the first and second optical refraction elements 11, 12. In the case of the first preferred embodiment, said display system 20 is integral in rotation with the binocular refraction test unit 10.

Moreover, in both embodiments, said binocular refraction test unit 10 is preferably mobile between at least two test configurations: a horizontal vision test configuration where the first and second optical refraction elements 11, 12 are positioned in a first position where said first and second optical axes OA1, OA2 extend horizontally and an inclined vision test configuration where the first and second optical refraction elements 11, 12 are inclined compared to said first position, so that said first and second optical axes OA1, OA2 are inclined downwards.

The inclined vision test configuration is preferably used to determine refraction features of the eyes of the subject in near vision condition.

The horizontal vision test configuration may be used to determine the refraction features of the eyes in near and/or intermediary vision conditions, but is preferably used to determine the refraction features of the eyes in far vision conditions.

In order to display a visual target in far vision conditions, the display system 20 of the first embodiments described here may have optical components for displaying test images at a variable distance from the refraction test unit, as mentioned before. Alternatively, the display system 20 described here and used for near and/or intermediary vision tests may be a removable display unit. The display system 20 is then removed from the refraction test unit 10 and an additional display device may be used to display the target in far vision conditions.

In the case of the second embodiment, an additional display device may be used to display the target in far vision conditions.

According to a first possibility, said horizontal rotation axis of the refraction test unit 10 goes through the common support 13 of said optical refraction elements 11, 12, above them. It coincides for example with the longitudinal axis H of the support 13 of the refraction test unit. In this case, the binocular refraction test unit 10 is also mobile in translation along a vertical direction.

The movement of the optical refraction unit 10 in front of the eye of the subject in this case is shown on figures 4 to 6.

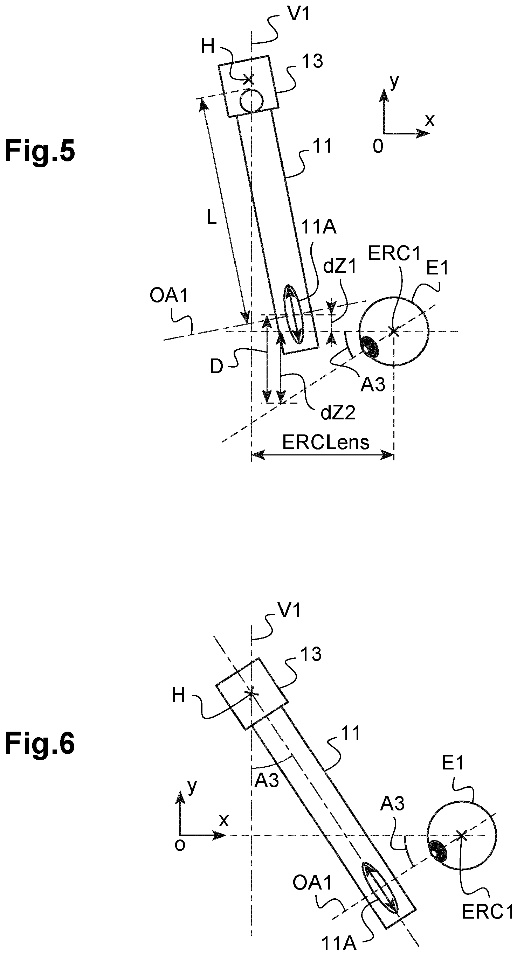

When going from the horizontal vision configuration (figure 4) to the inclined vision configuration (figure 6) of the refraction test unit, the refraction test unit 10 is pivoted towards the eyes E1, E2 of the subject (figure 5), then moved vertically downwards. The optical refraction element 11 of figure 5 is shown in an intermediary vision position where the refraction test unit is pivoted at an intermediary vision angle with no downwards translation.

The downwards movement of the refraction test unit 10 is along a vertical distance D (figure 5) depending on the final angle of inclination A3 of the refraction test unit relative to the vertical direction, the distance L between the optical axis OA1 of the optical refraction element 11, 12 and the horizontal rotation axis H of the refraction test unit 10 and the distance ERCLens between the center of rotation of the eye ERC1 and the center of the set of optical components of the corresponding optical refraction element 11 (figures 4 and 5). The angle of inclination A3 corresponds here to the angle AD defined on figures 2 and 3.

The distance D is equal to the sum of dZ1 and dZ2 as shown on figure 5.

The distance dZ1 is the vertical distance between the center of the set of optical components 11A, 12A of the optical refraction element 11 when it has been pivoted by angle A3 and the center of the set of optical components 11A, 12A of the optical refraction element 11 when it is oriented vertically.

The distance dZ2 is the vertical distance between the center of the set of optical components 11A, 12A of the optical refraction element 11 when it is oriented vertically and the gaze direction of the corresponding eye of the subject when this gaze direction is inclined downwards by angle A3.

The distances dZ1 and dZ2 may be calculated with the following formula:

$$dZ1=L*(sin(A3))^2$$

$$dZ2=ERClens*sin(A3).$$

The refraction test unit is then moved downwards along a vertical direction by a distance D = $L*(sin(A3))^2$+ ERClens*sin(A3).

The refraction test unit should also preferably be moved along the x axis of a translation dx=L*sin(A3)-ERClens(1-cos(A3)) order to maintain a similar distance between the eye and the set of optical components of the optical refraction elements 11, 12.

Based on this calculation, an automatic adjustment of the refraction test unit 10 can be done. With a motorized refraction test unit, the optometry device can be programmed to adjust automatically the position of the refraction test unit 10.

When the refraction test unit 10 is used in the inclined configuration, in near and/or intermediary vision conditions, the horizontal distance PD between the optical refraction elements 11, 12 is preferably reduced. The amount dPD of reduction is: dPD=PD*(1-Dist/(Dist+ERCLens)) with: Dist the distance between the test image displayed and the optical center of the set of optical components of the optical refraction element, and ERCLens the distance between eye rotation center and the center of the set of optical components of the optical refraction element (typically 25.5 to 27 millimeters (mm)).

Based on this calculation, an automatic adjustment of the horizontal distance between the two optical refraction elements 11, 12 of the refraction test unit 10 can be done to take into account the convergence of the eyes in near and/or intermediary vision conditions.

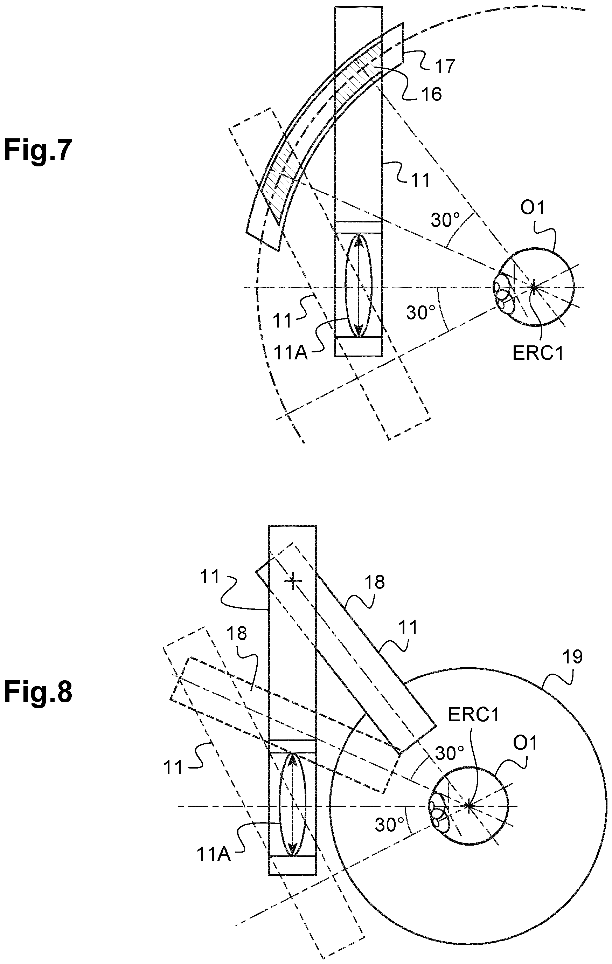

Preferably, said horizontal rotation axis about which said binocular refraction test unit 10 is mobile in rotation goes through a predetermined point located where a center of rotation ERC1 of one of the eyes E1 of the subject is situated when the subject's eyes look through the refraction test elements (figures 7 and 8). Preferably, as mentioned before, the axes V1, V2 about which each optical refraction element may pivot also go through said predetermined point located where a center of rotation ERC1 of one of the eyes E1 of the subject is situated when the subject's eyes look through the refraction test elements.

In practice, said horizontal rotation axis about which said binocular refraction test unit 10 is mobile in rotation goes preferably through two predetermined points located where the centers of rotation ERC1 of the eyes E1, E2 of the subject are situated when the subject's eyes look through the refraction test elements 11, 12.

Two possible structures are schematically represented on figures 7 and 8. Each of these figures shows a side schematic view of the binocular refraction test unit 10 in front of the eyes O1 of the subject.

In the first structure of figure 7, the binocular refraction test unit 10 is provided with a pin 16 extending laterally and received in a circular groove 17. This circular groove 17 is for example provided in a vertical panel of said supporting structure of the binocular refraction test unit 10. The circular groove 17 is centered on a point belonging to a horizontal axis going through the expected location of the centers of rotation of the eyes E1, E2 of the subject. The positions of these centers of rotation are for example controlled thanks to a device for receiving the head of the subject, for example his chin and forefront. Thanks to this arrangement, the binocular refraction test unit 10 may be pivoted about said horizontal axis going through the centers of rotation of the eyes E1, E2. Figure 7 shows the binocular refraction test unit 10 in its horizontal vision test configuration in full line. It shows in dashed lines its inclined vision test configuration. Here the refraction test unit is rotated by 30 degrees providing a downward gaze direction for the subject, inclined by 30 degrees below the horizontal direction.

In the second structure of figure 8, the refraction test unit 10 is integral with an arm 18 linking the refraction test unit 10 to a rotating plate 19. The rotating plate 19 is for example provided as part of said supporting structure of the refraction test unit 10. The rotating plate 19 rotates about a horizontal axis going through the expected location of the centers of rotation of the eyes E1, E2 of the subject. The position of these centers of rotation is for example controlled thanks to a device for receiving the head of the subject, for example his chin and forefront.

Thanks to this arrangement, the optical refraction unit may be pivoted about said horizontal axis going through the centers of rotation ERC1 of the eyes E1, E2. Figure 8 shows the refraction test unit 10 in its horizontal vision test configuration in full line. It shows in dashed lines its inclined vision test configuration. Here the refraction test unit is rotated by 30 degrees providing a downward gaze direction for the subject, inclined by 30 degrees below the horizontal direction.

Thanks to any of these examples of structure, going from the horizontal vision configuration to the inclined vision configuration of the refraction test unit is very easy as the eyes remain aligned with the optical components of each optical refraction element such as the variable lens. No additional adjustment is necessary.

Moreover, in the case where the display system 20 is integral in rotation with the refraction test unit 10, the relative position of these two parts of the optometry device being fixed, no further adjustment is necessary either.

As mentioned before, in order for the binocular measurement of the refraction features of the eyes to be accurate, the two test images provided by the display system 20 to the eyes of the subject have to be accurately positioned in order to appear as perfectly overlapped as possible for the subject.

The mobility of the two optical refraction elements 11, 12 allows them to be moved in their mean plane and about the axes V1, V2 (figures 4, 11) to adjust the distance between the sets of optical components of the optical refraction elements 11, 12 depending on the interpupillary distance of the subject and the orientation of these sets of optical components depending on his convergence in near and/or intermediary vision conditions.

Moreover, in the first embodiment where the refraction test unit 10 and the display system 20 are integral with each other, the alignment of the two parts of the optometry device 1 is fixed and accurate. In the case of the second embodiment, this alignment is adjusted each time one of the refraction test unit 10 or display system 20 is moved.

In addition to these possible accurate positioning of the parts of the optometry device 1, 2 in front of the eyes of the subject, the position of said first and second test images within said display system is adjustable in order for said first and second test images to be aligned with said first or second optical pathways.

In practice, said first and second test images are for example provided on one or two screens, as described in more details later.

Each screen may comprise an active screen or a passive screen associated with a projector.

The active screen may thus comprise an LCD screen, an OLED screen or a microdisplay comprising pixels. The passive screen may also comprise a surface on which a test image may be projected. It may for example comprise a holographic film.

The position of each test image may then be adjusted on the screen, in both directions, pixel by pixel, to ensure accurate relative positioning of the two test images and of the test images relative to the eyes of the subject. The test images may then be accurately overlaid to ensure appropriate fusion by the brain of the subject.

This adjustment may be performed for each subject or may be performed in a calibration step prior to using the optometry device, without having to perform this calibration step for each subject.

The optometry device 1, 2 also preferably comprises an alignment verification device for checking the alignment of said first and second optical pathways OP1, OP2 with each eye E1, E2 of the subject, comprising at least a camera oriented towards the eyes of the subject or an eyetracking device.

The camera is preferably a near InfraRed camera, so as to easily capture eye features such as pupil position.

In particular, the alignment verification device may comprise a retractable camera 28 (as shown on figure 13), adapted to be placed in front of the subject, for example in front of the middle of one of the screens, for verifying the alignment and removed when the eyes are tested. The camera, when activated, has indeed preferably an optical axis close to the center of the test image, so as to avoid parallax error when checking eye alignment. When not in use, the camera is retracted out of the optical path of the light emitted by the screens.

The alignment verification device may also comprise an eye tracking device or a sensor. The eye tracking device may be fixed on the refraction test unit 10 near the eyes, for example close to the set of optical components 11A, 12A. The sensor may be integrated to one of the screens of the display system 20 or added to the display system 20. The data generated by the eye tracking device or sensor may give indications from which the orientation of the eyes and gaze directions may be deduced. Data from the sensor may also be used to determine the distance between the eye and the corresponding test image or screen. The alignment verification device may also comprise a sensor attached to the refraction test unit for measuring the inclination of the refraction test unit.

The eye alignment verification can be done by comparing the position of the center of eye pupils with the center of the refraction test unit optics or the optical axis of each of the optical refraction elements 11, 12. The optical axis or center of the optics and the eye pupil positions can be determined from image processing, by detecting for example round edges of the optics and the pupil circular shape. Alternatively, instead of detecting the round edge of the refraction test unit optics, a known pattern can be used, for example identifying a small printed cross at the center of the lenses of the optical refraction test elements.

Finally, the camera may also be used to determine near and/or intermediary vision distance. Elements of the refraction test unit having a known size (printings, edge of phoropter optics...) may be identified on the image captured by the camera. The distance from the camera to the refraction test unit 10 may then be derived from the pixel size of the images of these elements.

Regarding now the image display system 20, regardless of the embodiment of the optometry device considered, it comprises at least one screen and one zone for displaying said first and second images as described in details hereafter.

More precisely, we will describe hereafter different examples of implementation of the display system 20, as illustrated on figures 9 to 16.

In a general manner, in the different examples of implementation described here, the two test images are produced on either two different, separate screens S1, S2, as it is the case in figures 9, or on a single screen S0, as shown for example on figures 10, 14, 15 and 17.

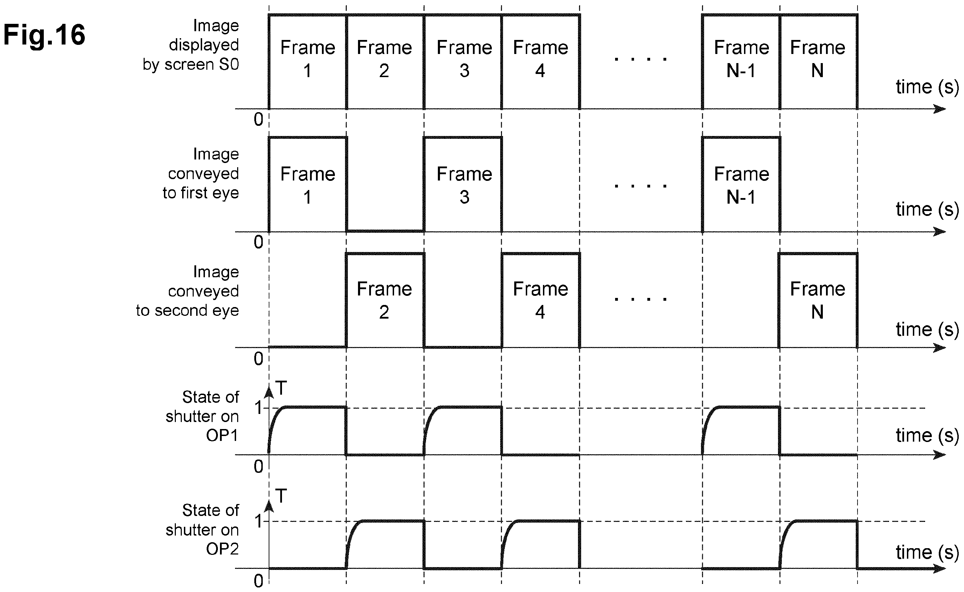

Moreover, the two test images may be produced in two different, separated display zones, belonging to two different, separate screens S1, S2 (figure 9) or to a single screen S0 (figure 10), or may be produced in a single display zone of a single screen S0 (figures 16, 15 and 17).

In a general manner, in the case where said display system 20 produces said first and second test images I11, I12, I21, I22 in two separated display zones, said optometry device 1, 2 comprises a light conveying unit conveying said first and second images to said first and second eye E1, E2 along said first and second pathways OP1, OP2.

In the case where each test image is produced by a different, separate screen S1, S2 as shown on figure 9, said light conveying unit may comprise a beam splitter and/or a mobile polarizer and/or a switchable polarizer and/or a wavelength filter.

In the first example of implementation of the display system 20 shown on figure 9, its optical components are housed in said box 21.

It comprises two screens S1, S2. This box 21 is provided with a large opening 22 allowing the subject to see all of each screen S1, S2.

In the first example of implementation of figure 9, the two screens S1, S2 are oriented perpendicularly to each other and said light conveying unit comprises a beam splitter 31.

The beam splitter 31 is preferably a polarization dependent beam splitter, and the screens S1, S2 emit preferably polarized light.

The polarization dependent beam splitter 31 separates an incident beam of arbitrary polarization in two polarized beams: one is transmitted with a first linear polarization and the other is reflected with a second linear polarization perpendicular to the first one. If the incident beam is already linearly polarized along the first or second linear polarization of the beam splitter, it is completely transmitted or reflected.

The polarizations of the light emitted by the two screens are here orthogonal and correspond to the first and second linear polarizations of the beam splitter, as shown on figure 9. Moreover, the optical refraction elements 11, 12 of the refraction test unit 10 preferably include polarizers oriented in perpendicular directions each corresponding to the direction of the polarization of the light emitted by a corresponding screen. In practice, it includes two orthogonal polarizers, each of them placed in front of one of the eyes E1, E2 of the subject.

The polarizers act as filters only allowing light having a predetermined linear polarization to go through the polarizer. All other polarizations of light are filtered out. By orthogonal polarizer or polarizers oriented in perpendicular directions, it is meant that the polarizations allowed by the two polarizers are oriented in perpendicular directions.

As shown schematically on figure 9, the light emitted by the first screen S1 is transmitted on the first optical path OP1 by the beam splitter 31 without change of polarization. It arrives to a first optical refraction element 11 with a polarization parallel to the polarization of this first optical refraction element 11 and is thus transmitted to the first eye E1 of the subject through the optical refraction element 11.

The light emitted by the second screen S2 is reflected on the second optical path OP2 by the beam splitter 31 without change of polarization. The light arrives to the second optical refraction element 11 with a polarization parallel to the polarization of this second optical refraction element 12 (orthogonal to the polarization of the first optical refraction element 11) and is thus transmitted to the second eye E2 of the subject through the optical refraction element 12.

Thanks to the orthogonal polarization of the light conveyed on the first and second optical path and the corresponding polarization of the optical refraction elements, each eye of the subject only sees one of the two images displayed by the display system 20. Moreover, the intensity of the light transmitted to the eyes is higher than it would be if the same setting was used with a polarization independent beam splitter and non polarized screens.

In practice, this first example of implementation may be achieved as shown on figures 11 to 13.

In order for the subject to be able to see in binocular vision the tests images, his brain must combine the two test images seen by the two eyes E1, E2. To this end, as mentioned before, the position and orientation of the screens, the position of the test images on the screens, and the position of the beam splitter must be well adjusted.

The mechanical design shown on figure 11, 12 and 13 allows controlling precisely the relative positions of the different elements.

The display system 20 comprises here a square or rectangular plate 23 on which is mounted two reception slots 24 perpendicular to the plate 23.

The two reception slots 24 are oriented at right angle in a corner of the plate 23. They are each adapted to receive a screen S1, for example adapted to receive a smartphone. The slot 24 is adapted to fit the screen closely in order for it to be blocked in a fixed position. In a diagonal of the plate passing through said corner, the beam splitter is received in grooves 25 blocking it in a fixed position, perpendicular to the plate and at 45° of each screen.

Each of the reception slots 24 and grooves 25 comprises for example at least one tightening screw to hole the screen or beam splitter.

As shown on figure 13, the plate 23 with fixed screen and beam splitter is housed in the box 21 mounted on a rail 26. The box 21 may be fixed, thanks to tightening screws 27 at different positions along the rail 26.

As shown on figures 1, 2 and 13, the rail 26 is attached on the refraction test unit 10. It may be a removable attachment or a permanent attachment.

In a variant, said screens and/or said beam splitter and/or said optical refraction elements may be polarization independent. Additional polarizers may be added in front of the screens and in front of the optical refraction elements. In another variant, said screens and said beam splitter are polarization independent.

In a variant, the display unit comprises one polarization rotator which rotates alternatively in front said first and second test images, and the first and the second optical refraction elements comprises respectively a first polarizer and a second polarizer with a different axis of polarization from the first polarizers. In this case, the polarization rotator may be disposed on the pathways between the images and the optical refraction elements in order to rotate alternatively the polarization axis on the first optical pathway and on the second optical pathway. For example, the polarizer may be disposed on the screens. In such manner, according to the rotation of the polarization rotator, the first test image will be seen by the first eye then the second eye will be seen by the second eye. The frequency of the rotation of the polarization rotator may be at least equal to 60 Hz.

The polarization rotator may be a faraday rotator, birefringent rotator such as an electronic liquid crystal polarizer, or a prism rotator.

In other example of implementation, not illustrated on the figures, the conveying unit may also comprise a filter, adapted to transmit and/or reflect selectively light having a wavelength comprised in a predetermined wavelength range. The first test image 111, 121 produced by the first screen S1 is emitted with a first set of wavelengths, for example 440, 550 and 600 nanometers, and the second test image 112, 122 produced by the second screen S2 is emitted with a second set of wavelengths, for example shifted by 20 nanometers as compared to the first one, for example 460, 570 and 620 nanometers. Two trichromic filters of corresponding wavelengths are placed on said first and second pathways. Holographic or any other appropriate technology known from the man skilled in the art may be used to obtain such filters.

As shown on figure 10, in a second example of implementation where each test image is produced by a single screen S0 divided in two display zones Z1, Z2, an unused zone Z0 displaying no image is preferably located in between the two display zones Z1, Z2.

This unused zone Z0 separates the two display zones Z1, Z2 and helps keeping the images seen by each eye of the subject separated.

Moreover, to ensure that each eye of the subject sees only the corresponding associated image, said light conveying unit may comprise a wall 30 blocking the transmission of light between said first and second optical pathways OP1, OP2, as it is the case in the second example of implementation of the display system represented on figure 10.

In this case, the optical refraction elements 11, 12 are tilted and provide not only the spherical/cylindrical powers, but also some prismatic power. The optical components providing the prismatic power act on the light emitted by the two display zones of the screen to bring it to the eyes of the subject. The prismatic value depends on the near and/or intermediary vision distance, and can be calculated so that eyes converge at a given distance.

In a variant, said light conveying unit may also comprise two optical guides, each conveying the light emitted at one of said separated display zones Z1, Z2 towards one of the eyes E1, E2 of the subject.

In another example of implementation, said display system may also comprise two separate microdisplays, each of said separate microdisplays displaying one of said first and second images said light conveying unit comprises two optical guides, each conveying the light emitted at one of said microdisplays towards one of the eyes E1, E2 of the subject. Each microdisplay may be using microLED or microOLED technology. Each microdisplay could be as small as 10 millimeters wide, and placed in front of one of the eyes. A mechanical or optical device could change the distance between the microdisplays to simulate far or near vision conditions.

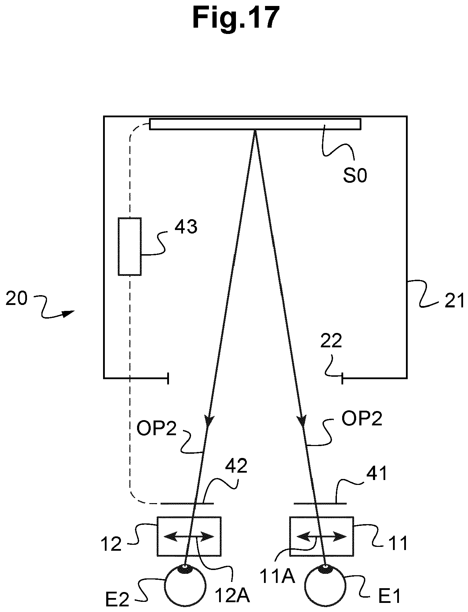

In the case where the two test images 111, 112, 121, I22 are produced in the same single display zone, the two test images may be produced either simultaneously in said single display zone (figures 14, 15) or they may be produced in an alternating manner (figures 16, 17).

In either case, the display system 20 comprises an image selection unit for selectively conveying said first and second test images to the first or second optical refraction element 11, 12 along said first or second optical pathway OP1, OP2. In other words, the display system 20 unit allows conveying the first test image 111, 121 to the first eye E1 of the subject and the second test image 112, I22 to the second eye E2 of the subject.

In a third example of implementation, the two test images are for example displayed simultaneously on the same display zone of the single screen S0 in an interlaced manner. In practice, each of the two test images is broken down into several parts that are displayed using separate parts of the single screen. By alternating the parts of each test images, and displaying these parts on parts of the screen spread onto the surface of the screen, the subject may perceived each of the two test images as displayed by the whole surface of the screen.

Each test image is for example displayed on groups of pixels of the screen. The pixels may for example be grouped by rows, columns, on a square or hexagonal elementary surface. The groups of pixels used may also be defined according to the colors of the pixel, using for example a Bayer's matrix. The first test image is displayed using some of the groups of pixels, and the second test image is displayed using the other groups.

For example, as shown on figures 14 or 15, the two test images are displayed using every other columns or rows of pixels of the screen S0. In other words, each test image is broken down into parts of the size of a column or row of pixels and the parts are displayed by alternating a column or a row of the first test image and a column or a row of the second test image. Out of every two columns, one column is used to display the first test image, and the other column is used to display the second test image. Similarly, out of every two rows, one row is used to display the first test image, and the other row is used to display the second test image.

The first test image 111, 121 is for example displayed using the first column of pixel of the screen and the columns of odd number, whereas the second test image 112, I22 is displayed using the second column of pixels and the columns of even numbers (figures 14, 15).

Thanks to the image selection unit, the first eye E1 of the subject only sees the first test image 111, 121, without seeing the second test image 112, I22, and the second eye E2 of the subject only sees the second test image 112, I22, without seeing the first test image 111, I21.

Said image selection unit may comprise a plurality of microlenses, each of them mapped with a part of the single display zone and adapted to convey the light emitted by this part of the single display zone in at least two different directions. A distance between the microlenses and the pixels of the screen is approximately equal to the focal length of the microlenses.

A microlens is for example associated with pairs of two adjacent groups of pixels defined above, each displaying a part of a different test image.

In the example described here, where the two test images are produced using every other columns of the screen S0, the microlenses could cover two adjacent columns and be adapted to convey the light emitted by one of the two columns to the first eye E1, and the light emitted by the other column to the second eye E2 (figure 15).

The microlenses used for different pairs of groups of pixels may be identical or different. They may comprise microlenses of different focal length or microlenses of different natures, for example comprising or not micro prisms.

According to a variant, the image selection unit may comprise a filter, adapted to transmit and/or reflect selectively light having a wavelength comprised in a predetermined wavelength range. The first test image 111, 121 produced by odd columns of the screen is emitted with a first set of wavelengths, for example 440, 550 and 600 nanometers, and the second test image 112, I22 produced by even columns of the screen is emitted with a second set of wavelengths, for example shifted by 20 nanometers as compared to the first one, for example 460, 570 and 620 nanometers. Two trichromic filters of corresponding wavelengths are used in front of the screen S0. The screen S0 or the filters may be mounted on a moving plate moving in translation, parallel to the filters or screen, back and forth with a high frequency. Holographic or any other appropriate technology known from the man skilled in the art may be used to obtain such filters.

The image selection unit may alternatively comprise a polarizer, adapted to transmit and/or reflect selectively light having a polarization oriented along a predetermined axis. The odd and even columns of the screen S0 may then be configured to emit light having different polarizations, in particular polarizations that are orthogonal to each other.).

Another possibility is that the two test images 111, 112, 121, I22 are produced in the same single display zone of said single screen S0 in an alternating manner, that is to say one after the other, using all the display zone of the screen. In the fourth example of implementation of the display system 20, each test image is displayed alternatively at a high frequency while a synchronized electronic shutter 41, 42 is blocking completely or at least partially the optical path for the eye to which the test image should not be conveyed (figure 17).

The single screen may be a Liquid Crystal Display (LCD), a screen using Organic Light-Emitting Diodes (OLED) or microLED or any other adapted screen known from the man skilled in the art.

In this case, said image selection unit comprises two shutters 41, 42, each of them being associated with one of said first and second optical pathways OP1, OP2 and presenting two states: an activated state in which the shutter blocks selectively the propagation of light in the corresponding first or second optical pathway, and a deactivated state in which said shutter allow selectively the propagation of light in the same first or second optical pathway.

The display device 20 then also comprises a synchronization device 43 to synchronize the change of state of both shutters and the production of the first and second test images. This way, when the test image for the first eye E1 of the subject is displayed, the shutter 41 placed on the first optical pathway OP1 is in its deactivated state and the shutter 42 placed on the second optical pathway OP2 is in its activated state. When the test image for the second eye E2 of the subject is displayed, the shutter 42 placed on the second optical pathway OP2 is in its deactivated state and the shutter 41 placed on the first optical pathway OP1 is in its activated state.

Each shutter 41, 42 comprises for example an electronic shutter associated with the display system 20 and/or with the refraction test unit 10.

Each electronic shutter 41, 42 may comprise for example an LCD screen.

Alternatively, the shutter may only partially block the light. It may comprise an active diffuser such as a component comprising a Polymer Dispersed Liquid Crystal layer.

Each LCD screen is placed on one of the first and second optical pathways OP1, OP2. It is placed preferably close to the eye of the subject, for example mounted on the refraction test unit 10. The activated state corresponds to the state where the LCD screen is opaque (black) and blocks entirely the propagation of light, and the deactivated state corresponds to the state where the LCD screen is transparent, therefore allowing the light to go through the LCD screen (figure 17).

The synchronization of the change of state of both shutters and the production of the first and second test images is represented schematically on figure 16.

The upper graph of this figure shows schematically the successive test images displayed by the single screen S0, corresponding to successive frames of a video for example. Each test image is displayed for the same period of time.

The two bottom graphs of this figure show the optical transmission T of each shutter placed on the first and second optical pathways OP1, OP2. As shown on the graphs, when the first test image is displayed, the shutter 41 placed on the first optical pathway OP1 allows transmission of the light, whereas the shutter 42 placed on the second optical pathway OP2 blocks the light. Correspondingly, the first test image is conveyed to the first eye E1, as seen on the second graph, whereas no image is conveyed to the second eye E2, as shown on the third graph.

The situation is reversed while the second test image ("frame 2") is displayed by the screen S0: the shutter 42 placed on the second optical pathway OP2 allows transmission of the light, whereas the shutter 41 placed on the first optical pathway OP1 blocks the light. Correspondingly, the second test image is conveyed to the second eye E2, as seen on the third graph, whereas no image is conveyed to the first eye E1, as shown on the second graph.

As shown on the two bottom graphs, the state of each shutter is changed whenever a new test image is displayed by the screen.

The three top graphs of figure 16 illustrating the synchronization of the display system 20 does not take into account the fact that test images are not instantaneously displayed on the screen. The displaying and the erasing of each test image takes time. In order to optimize the display system, the duration of the displaying of a test image, as well as the duration of the erasing (afterglow) of a test image has to be minimum.

The screen used in this example is chosen with the following additional specifications:

- frequency of the screen must be at least equal to 60 Hz, if possible, 90 Hz, 120 Hz or 240 Hz.

- the afterglow of the test image on the screen must be minimal. The timing of afterglow of the test image is preferably lower than 1/5 of the duration of the image displaying. If possible, the duration of afterglow and duration of displaying an image is lower that 1/10 of the duration of the image displaying.

For example, for a frequency of F=60 Hz:

- the duration of displaying of each of the test images is T=(1/60)s=16.6 milliseconds (ms);
- the duration of the afterglow dAfterglow of each test image is then preferably below 3 ms as dAfterglow < T/5 = 16.6/5 = 3 ms. Preferably, the duration of the afterglow dAfterglow of each test image is below 1.66 ms as dAfterglow < T/10 = 16.6/10=1.66 ms.

For example, an adapted screen has the following specifications:

- horizontal and optionally vertical dimensions equal to or over 56mm;
- pixel dimension below 50 microns to measure 20/10th (-0.3 LogMar) visual acuity, or below 74 microns to measure 15/10th ((-0.2 LogMar) visual acuity at 40 centimeters;
- screen frequency of at least 60 Hz;
- afterglow duration below 3 milliseconds (ms).

Smartphones screens may be used for this purpose.

Other types of screens, such as screens of virtual reality head set, could also be used.

Shutters used may for example be X-FOS(G2) ® shutters from swedish LC-Tec ® company.

The synchronization device 43 of the display system 20 sends to each shutter a signal for it to change from the activated state to the deactivated state and back.

The synchronization device 43 may be achieved in different ways.

The synchronization device 43 may comprise a photoreceptor placed in front of a specific part of the screen S0.

The synchronization device 43 then also comprises a part of each test image displayed by said screen S0; Each test image to be displayed for a predetermined eye, for example the first eye E1 of the subject, includes a white zone displayed on said specific part of the screen S0 in front of which the photoreceptor is placed, and a black zone on all the test images to be displayed for the other eye, for example the second eye E2 of the subject.

When the test images with a black zone are displayed, the photoreceptor receives a signal completely different from the signal received when the test images with a white zone are displayed. The photoreceptor is connected to an electrical circuit to transform this signal into an input signal for the shutters.

Other synchronization devices using photoreceptors may be used such as a synchronization device using two different photoreceptors place in front of two different specific part of the screen S0. Each test image associated with one eye is then provided with a white zone positioned in a first of the two specific part of the screen, whereas the test images associated with the other eye are provided with a white zone positioned in the second specific part of the screen S0.

Alternatively a cable is for instance an USB cable connected to the main socket of the mobile phone 150. The use of such a cable is preferred because this solution has the advantage to be almost instantaneous.

Alternatively, when using for example the screen of a smartphone, said synchronization device may comprise the audio system of the smartphone, used to send an audio signal perfectly synchronized to the images. The audio signal is then connected to an electrical circuit to transform this signal into an input signal for the shutters 41, 42.

When the image for the first eye E1 is displayed, an electrical audio signal is generated. The electrical signal is sent to a micro-controller of the synchronization device 43. The micro-controller transforms this signal into an input signal for the shutters 41, 42.

When the shutters 41, 42 receive this signal, the shutter 41 placed on the first optical pathway OP1 is put in its deactivated state and the other shutter 42 is put in its activated state.

After a timing corresponding to the frequency F of the screen, the image for the second eye E2 is displayed, an electrical audio signal is generated. The electrical signal is sent to the micro-controller which transforms this signal into an input signal for the shutters 41, 42.

When the shutters 41, 42 receive this signal, the shutter 42 placed on the second optical pathway OP2 is put in its deactivated state and the other shutter 41 is put in its activated state.

The synchronization device 43 may comprise wireless communication means between the screen S0 and the shutters 41, 42, by Bluetooth, Wi-Fi or IR signal

The electronic shutter may also comprise two polarizers placed on each of said first and second optical pathways OP1, OP2. A first of the two polarizers has a fixed polarization and the orientation of the polarization of the other second polarizer may be controlled so that it can be alternatively placed parallel to the polarization of the first polarizer in the deactivated state or orthogonal to it in the activated state.

For example, said shutter may comprise an active polarization rotation plate placed on the screen S0 in front of it. This active polarization rotation plate has a first state in which a linear polarization of the screen is unchanged and a second state in which: the linear polarization of the screen S0 is tilted by 90°.

Two passive polarizers are then located on the refraction test unit 10, in front of each of the eyes of the subject, having orthogonal polarization axis, one parallel to the unchanged linear polarization of the screen and the other parallel to the linear polarization of the screen tilted by 90° by the active polarization rotation plate.

In this case, the synchronization device only synchronizes the active polarization plate with the screen S0.

An example of implementation of this display system comprises a screen with a linear polarization at 45°, is as for instance a smartphone screen.

The active polarization plate is positioned over the screen providing, when not activated, a rotation of the polarization of incident light by 90°. The polarization of the light emitted by the assembly of the screen and active polarization plate is then oriented at 135°. When activated, the rotation is null. The polarization of the light emitted by the assembly of the screen and active polarization plate is then oriented at 45°.

The active polarization plate is controlled by the smartphone, from the USB connector that provides both an activating/deactivating signal and power supply.

Alternatively, when said display system 20 produces said first and second test images alternatively in a single display zone, said image selection unit may comprise two filters, each adapted to transmit and/or reflect selectively light having a wavelength comprised in two separate predetermined wavelength ranges or two polarizers, adapted to transmit and/or reflect selectively light having different polarizations oriented along different predetermined axes.

One of the two filters or polarizers is placed in front of each eye of the subject. The two test images are alternatively displayed with wavelengths belonging to the two separate predetermined wavelength ranges of the filters or with polarizations parallel to said different predetermined axes.

Each eye only sees the test image associated to this eye. No synchronization is required. The optometry device described here can be used as visual stimulus during visual examination in near vision or intermediate vision.

The test images can be placed at a distance of near vision, 40 centimeters for example, or intermediate vision, 60 cm for example, of the eyes of the subject.

As presented in detail below, each of the first and second test images to be displayed comprises:

- a central part 111, 112; 121, 122 for displaying optotypes or any visual targets or remaining blank, and
- a peripheral part 131, 132; 141, 142 that surrounds the central part 111, 112; 121, 122 and contributes to a well balanced fusion process between the left and right visual pathways, for the subject.

The two images are stereoscopic images, designed to provide a 3 dimensional vision to the subject.

A tested-eye test image comprising optotypes or other visual targets in said central part is provided to said first eye of the subject. Meanwhile, a non-tested-eye test image also comprising optotypes or visual targets in said central part is here provided to the second eye of the subject.

The optotypes or other visual targets displayed in the central part of the non-tested-eye test image are displayed with a contrast that is lower than the contrast of the corresponding optotypes or visual targets of the tested-eye test image. The optotypes or other visual targets displayed in the central part of the non-tested-eye test image may be the same optotypes or visual targets as displayed in the tested-eye test image, only with a contrast that is lower than the contrast of the corresponding optotypes or visual targets of the tested-eye test image.

Then, the tested-eye test image with optotypes or other visual targets is provided to the second eye of the subject to determine refraction errors of this second eye, while the non-tested-eye test image with a lower contrast central part is provided to the first eye of the subject.

On the test images shown on figures 18 and 20, one of the test images, shown on the right side, has a lower contrast than the other test image of the same figure, shown on the left side.

Some subjects have a binocular vision that is strongly dominated by the image perceived by one of their eyes, called the dominant eye. In other words, such subjects have one visual pathway that dominates strongly over the other visual pathway, in the neural process of binocular image fusion. For such subjects, during the binocular refraction protocol described above, a "suppression" phenomenon may occur if a completely blank image is provided to the dominant eye of the subject, due to ocular rivalry between the left and right visual pathways of the subject. In this case, the image perceived by the subject is completely blank.

Such suppression of the optotypes, in the perceived image, makes of course the determination of the refraction error of the non-dominant eye difficult, or even impossible. Even if the ocular dominance of the subject is not strong enough to cause such suppression, it often causes blinking or flickering of the image perceived by the subject. Besides, during such a binocular refraction protocol, a flickering of the image perceived by the subject may also be caused by vision problems of the subject related to ocular vergence. These adverse effects make the binocular refraction protocol less accurate, or less comfortable for the subject, and longer to be carried on.

The peripheral part of the test images stabilizes the fusion of the two test images by the brain of the subject and reduces the "suppression" phenomenon. This way, the visual test is more comfortable for the subject and provides more accurate results.

Said test images 111, 112; 121, I22, or at least their peripheral parts 131, 132; 141, 142, show realistic activities usually carried out in near vision, such as cooking, reading...

Advantageously, as mentioned before, the test images provided to the eyes of the subject with said optometry device 1, 2 may have at least partially different contrast. For example, the tested-eye test image provided to the tested eye has a contrast of more than 80%, preferably more than 90%, preferably equal to 100%. The non-tested-eye test image provided to the other eye has a contrast of less than 15%; preferably less than 10%, preferably equal to 5%. In a general manner, the non-tested-eye test image provided to the other eye has a contrast of less than 50% of the contrast of the tested-eye test image provided to the tested eye.

The contrast may be defined as:
(Loptotype - Lbackground) / (Loptotype + Lbackground), Loptotype being the luminance of the optotype and Lbackground being the luminance of the background.

Alternatively, the peripheral part of the tested-eye test image provided to the tested eye and the peripheral part of the non-tested-eye test image provided to the other eye have the same contrast. Only the central part of the tested-eye test image and the central part of the non-tested-eye test image provided to the other eye have different contrasts. For example, the central part of the tested-eye test image has a contrast of more than 80%, preferably more than 90%, preferably equal to 100%. The central part of the non-tested-eye test image has a contrast of less than 15%; preferably less than 10%, preferably equal to 5%. In a general manner, the central part of the non-tested-eye test image has a contrast of less 50% of the contrast of the central part of the tested-eye test image.

Moreover, in order to improve the comfort of the subject and further reduce the suppression effect, the peripheral part of each test image comprises components having different binocular disparities.

In real life, because of the eyes' horizontal separation, the two eyes of the subject have a different point of view on any scene observed by the subject. The difference in the point of view of the eyes creates a binocular disparity that the brain uses to extract depth information from the combination of two two-dimensional retinal images.

Binocular disparity refers to the difference in position of an object seen by the left and right eyes during binocular observation of this object. In practice, it is representative of the distance between two corresponding points in the left and right image of a stereo pair, reflecting the difference in image location of an object seen by the left and right eyes resulting from the eyes' horizontal separation and the distance between the eyes of the subject and the object.

A similar difference in point of view is simulated in a stereoscopic 3D representation by creating two images differing from each other by a difference in point of view corresponding to a predetermined disparity, each of them being displayed to one of the eye of the subject.

The disparity of the stereoscopic 3D representation of any component of said test images is determined based on the actual distance between this object of the subject in real life and on the interpupillary distance of the subject. The disparity is typically larger for objects that are closer to the subject than the screen and are negative (crossed) for the objects that are closer than the screen and positive (uncrossed) for objects that are further away from the screen. It may be expressed as a visual angle under which the component is seen.

In the test images of the examples shown on figures 18 and 20, the components of the peripheral parts of the test images are fruits. Each type of fruit is displayed with a different disparity. For example, components of the peripheral part of the test images have disparities of 1600", 800", 400", 340", 280", 200" 160",120",100", 80". The disparities are here expressed as arc minute angles.

The central parts 111, 112; 121; 122 of the test images 111, 112; 121, I22 of the examples shown on figures 18 and 20 are visual targets for sphere test for the first eye and the second eye in one case (figure 18) and visual target for cylinder test for the first eye and for the second eye in the other case (figure 20).

In each case, the visual target 112, 122 of the second test image displayed for the non-tested eye is identical to the visual target 111, 121 of the first test image displayed for the tested eye, with a lower contrast.

The final images seen by the subject are shown on figure 19 in the case of the two test images of figure 18, and on figure 21 in the case of the two test images of figure 20.

Finally, the optometry device 1, 2 according to the invention also comprises a control unit such as a computer, programmed to display the test images with an appropriate timing.

The sets of test images for first and second eyes may be put in the memory of the control unit. The control unit may comprise the smartphones when the screens used belong to smartphones. The change from a test image to the next may be manually triggered by the operator of the optometry device, for example by clicking on the screens when he wants to change the image.

Alternatively, in a preferred embodiment, the control unit may comprise a smartphone different from the one or two smartphones used in the display system to provide screens.

All smartphones are connected to the same Wi-Fi network, and communicate by TCP-IP protocol. They could also communicate using NearbyAPI protocol.

The operator chooses which test he wants to conduct: determination of sphere, cylinder, axis, visual acuity for the left eye, the right eye or both eyes for example.

A message is sent to each smartphone of the display system 20 indicating which test is to be performed.

Each smartphone of the display system receives the message and opens a file with a list of all the test images corresponding to this test. Once this file opens, the first image of the list is displayed on the screen of the corresponding smartphone.

The operator can remain away from the wearer and from the test.

## Claims

1. Optometry device (1, 2) for binocular testing of the eyes of a subject, comprising:

   - a binocular refraction test unit (10) with a first optical refraction element (11) adapted to provide different vision correction powers along a first optical axis (OA1) and a second optical refraction element (12) adapted to provide different vision correction powers along a second optical axis (OA2);
   - a display system (20) for providing a first and a second test images (I11, 112; I21, I22), the first test image being conveyed to the first optical refraction element (11)) along a first optical pathway (OP1) and the second test image being conveyed to the second optical refraction (12) element along a second optical pathway (OP2);

   said first and second test image being provided with details smaller than 1 arc minute, over a field of view of at least 8° in horizontal directions, wherein said first and second optical pathways (OP1, OP2) have a length comprised between 25 and 100 centimeters, allowing testing the eyes of the subject in near and/or intermediary vision.

2. Optometry device (1, 2) according to claim 1, wherein said binocular refraction test unit (10) presents an inclined vision test configuration where the first and second optical axis (OA1, OA2) of the first and second refraction elements (11, 12) are inclined downwards.

3. Optometry device (1) according to any one of claims 1 and 2, wherein said display system (20) is mechanically linked to said binocular refraction test unit (10) to be integral with the binocular refraction test unit (10).

4. Optometry device according to any one of claims 1 to 3, wherein said binocular refraction test unit (10) is mobile between at least two test configurations: a horizontal vision test configuration where the first and second optical refraction elements (11, 12) are positioned in a first position where said first and second optical axis (OA1, OA2) extend horizontally and an inclined vision test configuration where the first and second optical refraction elements (11, 12) are inclined compared to said first position, so that said first and second optical axis (OA1, OA2) are inclined downwards.

5. Optometry device according to claim 4, wherein said binocular refraction test unit (10) is mobile as a whole in rotation about a horizontal axis (H) parallel to a mean plane (MP) of the first and second optical refraction elements (11, 12), said horizontal axis (H) going through a predetermined point located where a center of rotation (ERC1) of one of the eyes (E1, E2) of the subject is situated when the subject's eyes look through the refraction test elements.

6. Optometry device according to any one of claims 1 to 5, wherein the position of said first and second test images (111, 112, 121, I22) within said display system (20) is adjustable in order for said first and second images to be aligned with said first or second optical pathways (OP1, OP2).

7. Optometry device according to any one of claims 1 to 6, wherein said display system (20) comprises a single display zone where said first and second test images are both produced and an image selection unit for selectively conveying said first and second test images to the first or second optical refraction element (11, 12) along said first or second optical pathway (OP1, OP2).

8. Optometry device (1, 2) according to claim 7, wherein said image selection unit comprises a filter, adapted to transmit and/or reflect selectively light having a wavelength comprised in a predetermined wavelength range or a polarizer, adapted to transmit and/or reflect selectively light having a polarization oriented along a predetermined axis.

9. Optometry device (1, 2) according to any one of claims 7 and 8, wherein said display system (20) produces said first and second test images simultaneously in said display zone and said image selection unit comprises a plurality of microlenses (50), each of them mapped with a part of the single display zone and adapted to convey the light emitted by this part of the single display zone in at least two different directions.

10. Optometry device (1, 2) according to any one of claims 7 and 8, wherein:

    - said display system (20) produces said first and second test images alternatively in said single display zone and
    - said image selection unit comprises two shutters (41, 42), each of them being associated with one of said first and second optical pathways (OP1, OP2) and presenting two states: an activated state in which said shutter (41, 42) blocks selectively the propagation of light in the corresponding first or second optical pathway (OP1, OP2), and a deactivated state in which said shut-

31　　　　　**EP 4 082 422 A1**　　　　　32

ter (41, 42) allow selectively the propagation of light in the same first or second optical pathway (OP1, OP2),
- a synchronization device is provided to synchronize the change of state of both shutters (41, 42) and the production of the first and second test images (111, 112; 121, I22).

**11.** Optometry device (1, 2) according to claim 10, wherein each shutter (41, 42) comprises an electronic shutter associated with the display system (20) and/or with the refraction test unit (10).

**12.** Optometry device (1, 2) according to any one of claims 1 to 6, wherein said display system (20) produces said first and second test images in two separated display zones (Z1, Z2) and wherein said optometry device comprises a light conveying unit conveying said first and second images to said first and second eye along said first and second pathways.

**13.** Optometry device (1, 2) according to claim 12, wherein said light conveying unit comprises a polarizer rotator which rotates alternatively in front said first and second test images.

**14.** Optometry device (1, 2) according to any one of claims 12 and 13, wherein said display system (20) comprises two separate screens (S1, S2), each of said separate screens (S1, S2) displaying one of said first and second images and said light conveying unit comprises a beam splitter (31).

**15.** Optometry device according to any one of claims 12 to 14, wherein said display system comprises:

- two separate microdisplays, each of said separate microdisplays displaying one of said first and second images or
- one screen providing said two separated display zones, each of said separated display zones displaying one of said first and second images,
and said light conveying unit comprises two optical guides, each conveying the light emitted at one of said separated display zones towards one of the eye of the subject.

17

**Fig.1**

**Fig.2**

**Fig.3**

**Fig.4**

**Fig.5**

**Fig.6**

**Fig.7**

**Fig.8**

**Fig.9**

**Fig.10**

**Fig.14**

**Fig.15**

**Fig.11**

**Fig.12**

**Fig.13**

**Fig.16**

Image displayed by screen S0: Frame 1 | Frame 2 | Frame 3 | Frame 4 | ... | Frame N-1 | Frame N — time (s)

Image conveyed to first eye: Frame 1 | Frame 3 | ... | Frame N-1 — time (s)

Image conveyed to second eye: Frame 2 | Frame 4 | ... | Frame N — time (s)

State of shutter on OP1 — time (s)

State of shutter on OP2 — time (s)

# Fig.17

**Fig.19**

**Fig.21**

**Fig.18**

**Fig.20**

Europäisches
Patentamt

European
Patent Office

Office européen
des brevets

# EUROPEAN SEARCH REPORT

Application Number

EP 21 30 5547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/000328 A1 (NAUCHE MICHEL [FR] ET AL) 5 January 2017 (2017-01-05) <br> * paragraph [0041] * <br> * paragraphs [0065] - [0068] * <br> * paragraphs [0091] - [0093] * <br> * paragraphs [0071], [0073], [0076], [0079] * <br> * paragraph [0129] * <br> * figures 1-4 * <br> ----- | 1-9,12, 15 | INV. <br> A61B3/028 <br> A61B3/032 |
| X | US 2019/274539 A1 (NAUCHE MICHEL [FR] ET AL) 12 September 2019 (2019-09-12) <br> * paragraphs [0027], [0051] * <br> * paragraphs [0063] - [0076] * <br> * paragraph [0082] * <br> * figures 1-5 * <br> ----- | 1,3,6-8, 10-15 | |
| X | US 2009/244486 A1 (ODA TATEFUMI [JP]) 1 October 2009 (2009-10-01) <br> * paragraphs [0024] - [0032] * <br> * figures 1, 2 * <br> ----- | 1,8,12, 14,15 | TECHNICAL FIELDS SEARCHED (IPC) |
| A | US 2020/037869 A1 (HIRAYAMA YUKITO [JP]) 6 February 2020 (2020-02-06) <br> * paragraphs [0049], [0054] * <br> * figures 1, 4, 6 * <br> * paragraphs [0137] - [0141] * <br> * paragraphs [0123] - [0124] * <br> ----- | 1-15 | A61B |

The present search report has been drawn up for all claims

| Place of search | Date of completion of the search | Examiner |
|---|---|---|
| Munich | 6 October 2021 | Gärtner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 30 5547

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2021

| Patent document cited in search report | Publication date | Patent family member(s) | Publication date |
|---|---|---|---|
| US 2017000328 A1 | 05-01-2017 | CN 105848563 A | 10-08-2016 |
| | | EP 3082565 A1 | 26-10-2016 |
| | | FR 3014673 A1 | 19-06-2015 |
| | | US 2017000328 A1 | 05-01-2017 |
| | | WO 2015092233 A1 | 25-06-2015 |
| US 2019274539 A1 | 12-09-2019 | AU 2017329869 A1 | 11-04-2019 |
| | | BR 112019005239 A2 | 04-06-2019 |
| | | CA 3037258 A1 | 29-03-2018 |
| | | CN 109788896 A | 21-05-2019 |
| | | CN 113367648 A | 10-09-2021 |
| | | EP 3298952 A1 | 28-03-2018 |
| | | EP 3515285 A1 | 31-07-2019 |
| | | JP 2019528920 A | 17-10-2019 |
| | | KR 20190052111 A | 15-05-2019 |
| | | US 2019274539 A1 | 12-09-2019 |
| | | WO 2018054997 A1 | 29-03-2018 |
| US 2009244486 A1 | 01-10-2009 | JP 5079575 B2 | 21-11-2012 |
| | | JP 2009240727 A | 22-10-2009 |
| | | KR 20090104729 A | 06-10-2009 |
| | | US 2009244486 A1 | 01-10-2009 |
| US 2020037869 A1 | 06-02-2020 | CN 110786822 A | 14-02-2020 |
| | | EP 3607873 A1 | 12-02-2020 |
| | | JP 2020018715 A | 06-02-2020 |
| | | US 2020037869 A1 | 06-02-2020 |

EPO FORM P0459

For more details about this annex : see Official Journal of the European Patent Office, No. 12/82